# EUROPÄISCHE PATENTSCHRIFT

(11) **EP 2 858 605 B1**
(45) Veröffentlichungstag und Bekanntmachung des Hinweises auf die Patenterteilung: **05.10.2016**
(21) Anmeldenummer: 13728961.7
(22) Anmeldetag: 06.06.2013
(51) Int. Cl.: A61F 5/01

(54) **KNÖCHEL-FUSS-ORTHESE**
ANKLE-FOOT ORTHOSIS
ORTHESE PEDI-JAMBIERE

(30) Priorität: 12.06.2012 DE 102012011466
(43) Veröffentlichungstag der Anmeldung: 15.04.2015
(73) Patentinhaber: Otto Bock Healthcare GmbH, 37115 Duderstadt (DE)
(72) Erfinder: JOHNSSON, Jan, 60163 Norrköping (SE); SIEWERT, Gordon, 37077 Göttingen (DE); DREWITZ, Heiko, 37130 Gleichen (DE); KROLL-ORYWAHL, Olaf, 37083 Göttingen (DE); LÜRSSEN, Marcus, 37073 Göttingen (DE); SEGL, Maximilian, 37115 Duderstadt (DE); TÜTTEMANN, Markus, 45731 Waltrop (DE); LJUBIMIR, Boris, 70825 Korntal-Münchingen (DE); SCHIMEK, Norbert, 37339 Kirchworbis (DE); KEINER, Wolfgang, 37115 Duderstadt (DE); SCHMITT, Alexander, 34123 Kassel (DE)
(74) Vertreter: Stornebel, Kai
(86) Internationale Anmeldenummer: PCT/EP2013/001663
(87) Internationale Veröffentlichungsnummer: WO 2013/185894

(56) Entgegenhaltungen:
- WO-A1-2007/106017
- DE-T2- 60 315 698
- DE-U1- 29 924 933
- US-A1- 2009 105 624

## Beschreibung

Die Erfindung betrifft eine Knöchel-Fuß-Orthese mit einem Fußteil, das eine Sohle zur Aufnahme eines Fußes aufweist, und einem Schienenbeinteil, das sich im angelegten Zustand frontal an ein Schienbein anlegt und mit dem Fußteil über eine Feder verbunden ist.

Bei mangelnder Stabilität des Knöchelgelenks sowie nicht ausreichender Muskelkraft kann es sinnvoll und notwendig sein, eine Knöchel-Fuß-Orthese einzusetzen. Durch die Knöchel-Fuß-Orthesen wird der Fuß in einer Zuordnung zu dem Schienbein gehalten und verhindert eine ungewollte Dorsalflexion ebenso wie eine Plantarflexion, wenn der Fuß unbelastet ist. Beim Gehen unterstützt die Orthese den Patienten, indem eine dorsal flektierte Stellung beim Fersenauftritt beibehalten und ein Abdrücken in der terminalen Standphase ermöglicht wird. Ebenso wird eine ungewollte Pronation und Supination des Fußes vermieden.

Die DE 603 15 698 T2, die den nächsten Stand der Technik darstellt, betrifft eine Fußgelenk-Fußorthese mit einem strukturellen Rahmen, der eine Fußplatte, einen inneren Anteil und einen Fußgelenksanteil aufweist und sich in zwei frontale Stützelemente aufspaltet, die frontal zu dem Schienbein medial und lateral angeordnet sind. Über eine Befestigungsvorrichtung in Gestalt eines Klettverschlussriemens kann die Fußgelenk-Fußorthese an dem Patienten angelegt werden.

Die DE 299 24 933 U1 betrifft eine Unterschenkelorthese mit einer den Unterschenkel umgreifenden Unterschenkelmanschette, die gelenkig mit einer Fußmanschette verbunden ist. Eine Stützfeder, die mit der Unterschenkelmanschette und der Fußmanschette verbunden ist, ist ventral angeordnet und verläuft von der Unterseite des Fußteils in einem Winkel entlang der Unterschenkelmanschette auf deren posterioren Seite.

Die WO 2005/106017 A1 betrifft eine Unterschenkelmanschette mit einem Fußteil und einem medial angeordneten Unterschenkelteil, das sich über eine posterior verlaufende Schale an dem Unterschenkel abstützt. Das Unterschenkelteil ist mit dem Fußteil über eine medial verlaufende Feder verbunden.

Die US 2009/0105624 A1 betrifft eine Knöchel-Fußorthese mit einem schalenförmigen Fußteil, das den Fersenbereich umschließt. An den Fersenbereich schließt sich in Längserstreckung des Unterschenkelteils ein Federabschnitt an, der in ein Schienbeinteil übergeht.

Die WO 2007/106017 A1 betrifft eine Unterschenkelorthese, mit einer Fußplatte, die eine Auflage für die Unterseite eines Patientenfußes bildet. Eine Zunge erstreckt sich medial nach oben und hinten in einem Wesentlichen rechten Winkel zu der Fußplatte. Eine Feder ist an ihrem unteren Ende mit der Zunge und an ihrem oberen Ende mit einer Schiene verbunden, deren oberes Ende eine U-förmige, nach vorne offene Unterschenkelaufnahme ausbildet. Die Feder weist zwei im Wesentlichen parallele Federstäbe auf, die gebogen und hinter dem Knöchel entlang geführt sind.

Aufgabe der vorliegenden Erfindung ist es, eine Knöchel-Fuß-Orthese bereitzustellen, die eine hohe Dauerfunktionsfähigkeit sowie ein einfaches Einsteigen in den Schuh und ein leichtes Anlegen an den Patienten ermöglicht.

Erfindungsgemäß wird diese Aufgabe durch eine Knöchel-Fuß-Orthese mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die erfindungsgemäße Knöchel-Fuß-Orthese mit einem Fußteil, das eine Sohle zur Aufnahme eines Fußes aufweist, und einem Schienbeinteil, das sich im angelegten Zustand frontal an ein Schienbein anlegt und mit dem Fußteil über eine Feder verbunden ist, sieht vor, dass die Feder posterior zu einem Knöchelbereich einen Gelenkbereich ausbildet, der eine Verlagerung des Schienbeinteils relativ zu dem Fußteil in anterior-posterior-Richtung zulässt. Durch den Verlauf der Feder posterior zu einem Knöchelbereich, also dem Bereich, in dem die natürliche Gelenkachse des Knöchelgelenks liegt, wird gewährleistet, dass kein Federmaterial gegen den Knöchel oder das Schienbein drückt. Die Erfindung sieht vor, dass die Feder medial verlaufend angeordnet ist. Der mediale Verlauf der Feder verhindert, dass sich die Feder während des Gehens an Gegenständen verfängt, zudem ist eine optisch unauffällige Positionierung der Knöchel-Fuß-Orthese durch den medialen Verlauf der Feder gewährleistet. Weiterhin kann dadurch die Feder im Bereich des Fußgewölbes mit dem Fußteil verbunden werden, wodurch der Federverlauf im Übergangsbereich fließender als im lateralen Sohlenbereich erfolgen kann.

Der Gelenkbereich der Feder ist um eine Achse torsionsstabil ausgebildet, die von proximal nach distal verläuft, also im Wesentlichen einer vertikalen Richtung entspricht. Dadurch ist es möglich, dass die Orientierung des Fußes relativ zu dem Schienbein während des Gehens beibehalten wird und lediglich eine Federbewegung aufgrund des Gelenkbereiches in der Sagittalebene erfolgt, also um eine quer zur Gehrichtung verlaufende Achse.

Der Gelenkbereich ist vorteilhafterweise in medial-lateral-Richtung biegesteif ausgebildet, um einer Pronationsbewegung oder Supinationsbewegung des Fußes oder des Fußteils entgegenzuwirken.

Eine Ausgestaltung der Erfindung sieht vor, dass die Feder vom Fußteil medial nach innen und schräg nach dorsal oben verläuft, so dass sie bis hinter den Knöchelbereich ragt. Von dem Knöchelbereich verläuft sie dann schräg nach ventral oben. Der Verlauf der Feder sieht somit vor, dass bei einer Anlenkung der Feder im Bereich des Fußgewölbes ein unterer Federabschnitt schräg nach hinten verläuft, wobei die Vorderkante der Feder hinter der Gelenkachse liegt. Von dem weitesten posterior gelegenen Punkt der Feder verläuft dann ein oberer Abschnitt der Feder schräg nach vorn, um das Schienbeinteil abzustützen.

Zur Ausbildung des Gelenkbereiches ist vorgesehen, dass die Feder eine Verjüngung in anterior-posterior-Richtung aufweist, so dass aufgrund der verschlankten Ausgestaltung der Feder in dem Bereich mit verringertem Material eine definierte Zone bereitgestellt wird, die ein Nachgeben in anterior-posterior-Richtung erleichtert.

Um die Dorsalflexion und Plantarflexion zu erleichtern, kann die Feder in anterior-posterior-Richtung ein geringeres Widerstandsmoment als in medial-lateral-Richtung aufweisen. Dieses geringere Widerstandsmoment kann durch eine bestimmte geometrische Ausgestaltung der Feder realisiert werden, insbesondere im Gelenkbereich können unterschiedliche Widerstandsmomente in den jeweiligen Orientierungen vorgesehen sein.

Um die Verschwenkung in anterior-posterior-Richtung zu ermöglichen und gleichzeitig die Verschwenkung in medial-lateral-Richtung zu vermeiden, damit der Fuß auf den Fußteil in der eingestellten Orientierung verbleibt, kann auch vorgesehen sein, dass die Feder im Gelenkbereich ein Materialaufbau aufweist, der eine in anterior-posterior-Richtung höhere Elastizität als in medial-lateral-Richtung hat. Dies kann beispielsweise durch eine spezielle Materialwahl bei faserverstärkten Werkstoffen für die Feder erfolgen. So kann beispielsweise in medial-lateral-Richtung Karbon als Fasermaterial eingesetzt werden, was eine geringere Elastizität als beispielsweise Glasfaser, Aramidfaser oder Polyethylenfaser, die für die Steifigkeit bzw. Elastizität in anterior-posterior-Richtung verantwortlich sind, aufweist. Die unterschiedlichen Elastizitäten können daher nicht nur durch geometrische Variationen, sondern auch durch die entsprechenden Orientierungen der Faserverstärkungen innerhalb der Feder realisiert werden.

Das Schienbeinteil kann rinnenförmig oder schalenförmig ausgebildet und in der Medialebene biegesteif ausgeführt sein, so dass eine stabile Anlage des Schienbeins an dem Schienbeinteil ermöglicht wird. Durch die biegesteife Ausgestaltung in der Medialebene kann eine gute Kraftübertragung von dem Fußteil über die Feder auf das Schienbeinteil erfolgen, ohne dass es zu einer punktuellen Belastung im Schienbeinbereich führt.

Die Feder kann bezüglich einer Sagittalebene in Medialrichtung gewölbt ausgebildet sein, um sich von dem Fußteil medial nach innen zu erstrecken, um den sich medial zur kontralateralen Seite hervorstehenden Knöchel nicht zu berühren, sondern ihn sowohl medial als auch in posteriorer Richtung beabstandet zu umlaufen.

Das Schienbeinteil kann eine Polsterung aufweisen, um das Tragen der Orthese zu erleichtern. Die Polsterung kann an dem Schienbeinteil über Taschen an Vorsprüngen festgelegt sein. Ebenfalls ist es möglich, dass die Taschen medial und lateral an dem gesamten Schienbeinteil anliegen, so dass das Schienbeinteil zumindest an den Rändern vollständig in den Taschen aufgenommen wird. Zur Befestigung sowohl der Orthese an dem Bein als auch der Polsterung an dem Schienbeinteil können Befestigungselemente vorgesehen sein, die von lateral nach medial geführt sind, um das Anlegen und Befestigen zu erleichtern. Die Feder kann als Bogen ausgeführt und als separates Bauteil mit einem separaten Fußteil und einem separaten Schienbeinteil verbunden sein. Grundsätzlich ist es auch möglich, dass die Knöchel-Fuß-Orthese einteilig ausgebildet ist, so dass die Feder integraler Bestandteil sowohl des Fußteils als auch des Schienbeinteils ist. Die Feder selbst kann als separates Bauteil ausgebildet und über Steckverbindungen, gegebenenfalls durch eine formschlüssige Sicherung, oder eine Klebeverbindung mit den jeweiligen anschließenden Komponenten verbunden sein.

Weiterhin kann vorgesehen sein, dass die Feder im Knöchelbereich die geringste Steifigkeit gegen eine Biegung um eine Knöchelgelenksachse aufweist, wodurch eine Beweglichkeit in anterior-posterior-Richtung ermöglicht wird. Beim Gehen oder Stehen verändert sich der Krafteinleitungspunkt in den Fuß bzw. in das Fußteil je nach Bewegungsphase oder Gewichtsverlagerung. Die auf das Knöchelgelenk wirkenden Kräfte verursachen Momente um die jeweilige Gelenkachse, wobei bei gleichbleibender Kraft das wirksame Moment mit dem Abstand zur Gelenkachse steigt. Aufgrund der geometrischen Verhältnisse wird daher um die Knöchelgelenksachse das größte Moment aufgebracht, da der Krafteinleitungspunkt maximal von der Knöchelgelenkachse oder dem Gelenkbereich entfernt ist, während um die Fußlängsachse aufgrund der geometrischen Bedingungen nur ein geringes Moment auf den Knöchelbereich und damit auch auf den Gelenkbereich aufgebracht wird. Dadurch ist eine relativ steife Ausgestaltung gegen eine Supinationsbewegung oder Pronationsbewegung gegeben, da auch bei gleicher Kraft aufgrund des geringen Abstandes zum Gelenkbereich ein geringeres Moment wirksam ist, während eine relativ weiche und elastische Ausgestaltung für eine Plantarflexion und Dorsalflexion vorhanden ist, da eine größere Nachgiebigkeit aufgrund des größeren, wirksamen Momentes um den Gelenkbereich gegeben ist.

Nachfolgend werden Ausführungsbeispiele der Erfindung anhand der beigefügten Figuren näher erläutert. Es zeigen:
- Figur 1 -: eine Knöchel-Fuß-Orthese im schematischen Aufbau;
- Figur 2 -: eine Knöchel-Fuß-Orthese mit Polsterung; sowie
- Figur 3 -: eine Knöchel-Fuß-Orthese mit offenen Befestigungselementen.

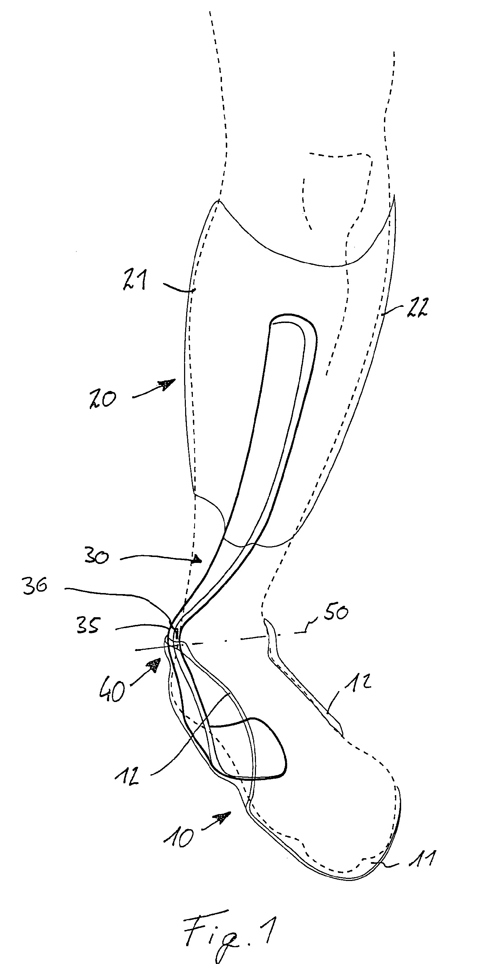

Figur 1 zeigt eine Knöchel-Fuß-Orthese in einer schematischen Darstellung mit einem Fußteil 10 und einem Schienbeinteil 20, die über eine Feder miteinander verbunden sind. Das Fußteil 10 verfügt über eine Sohle 11, auf der ein durch die Strichlinie angedeuteter Fuß positionierbar ist. An der Sohle 11 sind im Mittelfußbereich medial und lateral Erhöhungen vorgesehen, um den Fuß sowohl in medialer als auch in lateraler Richtung abzustützen. Die Erhöhungen 12 können auch die Ferse umfassen. Das Fußteil 10 ist vorteilhafterweise einstückig ausgebildet und besteht aus einem formstabilen, in geringem Umfang elastischen Material, das es erlaubt, eine Abrollbewegung des Fußes während des Gehens auszuführen. Die Stabilität ist dergestalt gewählt, dass eine ausreichende Abstützung des Fußes gewährleistet ist.

Das Schienbeinteil 20 ist schalenförmig oder rinnenförmig ausgebildet und folgt der Form eines natürlichen Beines. Das Schienbeinteil 20 kann in der Medialebene biegesteif ausgeführt sein, so dass eine feste Anlage entlang der Längserstreckung des Schienbeins erfolgt. Um Volumenschwankungen während des Tragens oder auch eine Anpassung an verschiedene Nutzer zu ermöglichen, kann das Schienbeinteil 20 in medialer und/oder lateraler Richtung an seinen Endbereichen elastisch ausgebildet sein, so dass sich das rinnenförmige Schienbeinteil 20 aufweiten lässt oder durch geeignete Befestigungselemente auf den Unterschenkel zu bewegen lassen kann. Ebenfalls ist es möglich, dass das Schienbeinteil 20 nur in einer Richtung, also entweder medial oder lateral elastisch ausgebildet ist, da es fallweise vorteilhaft ist, wenn eine Anlage steif ausgeführt ist, während die andere Anlage elastisch nachgiebig ist. Ist beispielsweise die mediale Anlage steif ausgeführt, kann eine Drehbewegung des Beins verhindert werden.

Beide Komponenten, sowohl das Schienbeinteil 20 als auch das Fußteil 10, können aus faserverstärkten Kunststoffen hergestellt sein und sind vorteilhafterweise einstückig ausgebildet. In einer alternativen Ausgestaltung kann auch eine mehrteilige Ausführung sowohl des Fußteils 10 als auch des Schienbeinteils 20 vorliegen.

Im dargestellten Ausführungsbeispiel ist das Fußteil 10 mit dem Schienbeinteil 20 über eine medial angeordnete Feder 30 verbunden. Fußteil 10, Schienbeinteil 20 und Feder 30 sind als separate Bauteile ausgebildet und aneinander befestigt, beispielsweise über eine Steckverbindung, die nachträglich fixiert wird, beispielsweise durch ein Formschlusselement oder durch eine Klebverbindung. Alternativ sind das Fußteil 10, das Schienbeinteil 20 und die Feder 30 dauerhaft miteinander verbunden, beispielsweise miteinander verklebt, verschweißt, verbacken oder einlaminiert. Grundsätzlich ist es auch möglich, sowohl das Fußteil 10 als auch die Feder 30 und das Schienbeinteil 20 einteilig aus faserverstärkten Kunststoffen oder einem anderen Material herzustellen.

Die Feder 30 verläuft medial von der Unterseite der Sohle 11 des Fußteils 10 nach proximal über den Fußgewölbebereich in Richtung auf den natürlichen Knöchel, wobei der Verlauf von der Sohle 11 bis zu einem Knöchelbereich 40 schräg nach posterior proximal verläuft, also nach schräg hinten oben, wobei die Vorderkante der Feder 30 bis hinter die natürliche Knöchelgelenksachse 50 verläuft. Von dort aus verläuft die Feder 30 weiter schräg nach anterior und proximal, also nach schräg oben vorn, bis sie im Wesentlichen parallel zum Schienbein proximal, also nach oben verläuft. An dem proximalen, oberen Ende der Feder 30 ist das Schienbeinteil 20 befestigt.

Die Feder 30 bildet im Knöchelbereich 40 der Knöchel-Fuß-Orthese einen Gelenkbereich 35 aus, der ungefähr auf der Höhe der Knöchelgelenksachse 50 liegt. In diesem Gelenkbereich 35 wird eine Biegung der Feder 30 im Wesentlichen um die natürliche Gelenkachse 50 herum ermöglicht, so dass das Schienbeinteil 20 elastisch relativ zu dem Fußteil 10 verlagert werden kann. Zur Ausbildung des Gelenkbereiches 35 ist es vorgesehen, dass sich der Querschnitt über den Verlauf der Feder 30 von distal zu proximal verändert und im Gelenkbereich 35 das geringste Widerstandsmoment gegen eine Bewegung in anterior-posterior-Richtung, also in der Sagittalebene hat. Dazu ist die Feder 30 im Gelenkbereich 35 ungefähr auf der Höhe der natürlichen Knöchelgelenksachse 50 mit einer Verjüngung 36 versehen, so dass in diesem Bereich das geringste Widerstandsmoment vorherrscht. Die Dicke der Feder 30, also die Erstreckung in medial-lateral-Richtung, kann dabei im Wesentlichen konstant ausgebildet sein.

Von der Sohle 11 verläuft die Feder 30 zunächst medial nach innen, um dann in einem Bogen hinter den Knöchel in Lateralrichtung zu verlaufen. Nach dem Gelenkbereich 35 verläuft die Feder 30 bogenförmig nach lateral, bis sie in das Schienbeinteil 30 mündet. Die Feder 30 verläuft somit in zwei Bögen, wobei die größte mediale Auswölbung unterhalb des Gelenkbereiches 35 oder im Gelenkbereich 35 liegt, während die maximale posteriore Auswölbung hinter dem Knöchelbereich 40 des Knöchelgelenks liegt.

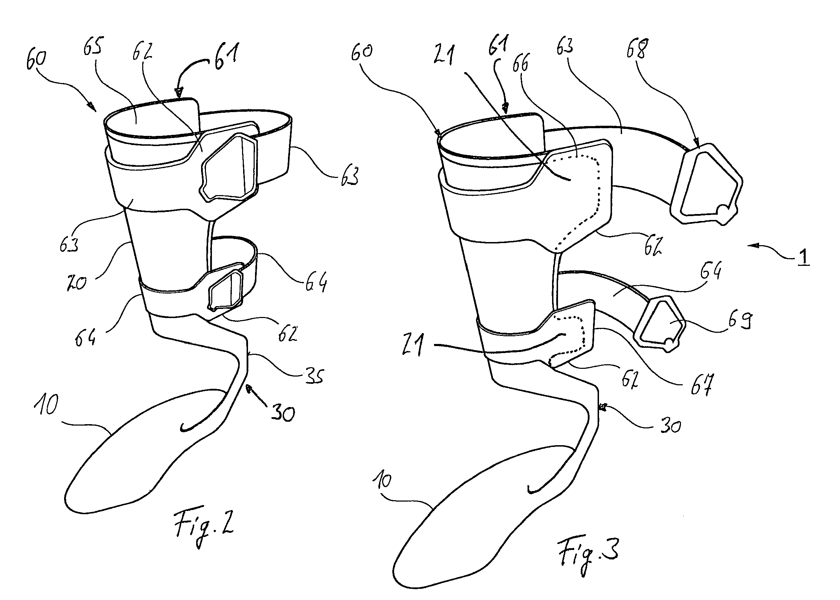

In der Figur 2 ist eine Variante der Knöchel-Fuß-Orthese dargestellt, mit einem Schienbeinteil 20, einem Befestigungsmittel 60 und einem Fußteil 10, das mit dem Schienbeinteil 20 über die Feder 30 verbunden ist. Die Feder 30 lässt dabei nur eine geringfügige, definierte Verlagerung des Schienbeinteils 20 relativ zu dem Fußteil 10 zulässt. Das Fußteil 10 ist als flache Sohlenplatte ausgebildet, die im Wesentlichen der äußeren Kontur eines Fußes oder eines Schuhs folgt. Das dargestellte Ausführungsbeispiel ist aus einem faserverstärkten Kunststoff einstückig ausgebildet.

Das Schienbeinteil 20 liegt im angelegten Zustand an dem Schienbein des Orthesennutzers an und umschließt das Schienbein medial und lateral zumindest teilweise. Die Kontur des Schienbeinteils 20 ist im Wesentlichen rinnenförmig oder schalenförmig und kann Ausnehmungen oder Durchbrechungen aufweisen, um eine verbesserte Belüftung des Schienbeinbereiches zu ermöglichen. Auf der Innenseite des Schienbeinteils 20, die im angelegten Zustand dem Orthesennutzer zugewandt ist, ist ein Befestigungsmittel 60 angeordnet, das gleichzeitig mit einer Polsterung 65 versehen ist, so dass es nicht zu einer direkten Anlage der harten, schalenartigen Konstruktion des Schienbeinteils 20 an dem Unterschenkel kommt.

An dem Schienbeinteil 20 sind sowohl medial als auch lateral Aufnahmebereiche 21 in Gestalt von Vorsprüngen oder Laschen angeordnet, die in der Figur 3 dargestellt sind. Die Aufnahmebereiche 21 oder Vorsprünge sind am proximalen Ende und distalen Ende des Schienbeinteils 20 ausgebildet und weisen im angelegten Zustand für den Orthesennutzer nach hinten. Nachfolgend wird von den Aufnahmebereichen 21 als Vorsprünge gesprochen, worunter auch eine Ausbildung zu verstehen ist, bei der an das Schienbeinteil 20 anschließende Ausformungen geeignet sind, in Taschen aufgenommen zu werden, um eine formschlüssige Festlegung der Polsterung 65 zu bewirken.

An dem Befestigungsmittel 60 sind Taschen 61, 62 ausgebildet, in die die Vorsprünge oder Aufnahmebereiche 21 eingeschoben sind. Durch das Einschieben der Vorsprünge oder Aufnahmebereiche 21 in die Taschen 61, 62 wird das Befestigungsmittel 60 an dem Schienbeinteil 20 festgelegt. Dabei ist vorgesehen, dass die lateralen Taschen 61 tiefer als die medialen Taschen 62 sind, ebenso ist vorgesehen, dass die lateralen Vorsprünge oder Aufnahmebereiche 21 länger als die medialen Vorsprünge oder Aufnahmebereiche 21 sind, wobei die Tiefe der Taschen 61, 62 zu den Längen der Vorsprünge 21 korrespondiert. Das Ausführungsbeispiel sieht medial und lateral jeweils zwei Taschen 61, 62 und zwei Vorsprünge 21 vor, die in Längserstreckung versetzt zueinander angeordnet sind, so dass sich zwei proximale und zwei distale Paarungen von Taschen 61, 62 und Vorsprüngen oder Aufnahmebereichen 21 ergeben. Alternativ kann auch nur jeweils eine Tasche medial und lateral vorgesehen und an einem korrespondierenden Aufnahmebereich 21 festgelegt sein.

An dem Befestigungsmittel 60 sind sowohl am proximalen Rand als auch am distalen Rand des Schienbeinteils 20 Befestigungselemente 63, 64 vorgesehen, über die eine Festlegung der Orthese an der Gliedmaße erfolgt. Die Befestigungselemente 63, 64 in Gestalt von Gurten oder Bändern sind auf der Medialseite an dem Befestigungsmittel 60 unter Ausbildung der medialen Taschen 62 befestigt, beispielsweise angeschweißt, angeklebt oder angenäht. Von der medialen Tasche 22 ist das als Gurt ausgeführte Verbindungselement 63, 64 außen auf der Außenseite des Schienbeinteils20 entlang geführt und außen an der lateralen Tasche 61 festgelegt, beispielsweise durch einen Klettverschluss. Bei einer elastischen Ausgestaltung des gurtartigen Befestigungselementes 63, 64 werden die medialen und lateralen Taschen 61, 62 in Umfangsrichtung aufeinander zu mit einer Vorspannung beaufschlagt, so dass die nach vorne offenen Taschen 61, 62 nicht von den nach hinten weisenden Vorsprüngen 21 heruntergleiten können. Zum weiteren Festlegen der Orthese wird dann das jeweilige Verbindungselement 63, 64 um die nicht dargestellte Gliedmaße hinten herum von lateral nach medial geführt und dort auf einer Fixiereinrichtung 66, 67 festgelegt. Das Verbindungselement 63, 64 ist somit einmal um die Orthese und die Gliedmaße herumgeführt, wobei das freie Ende auf der Außenseite der medialen Tasche 62 festgelegt wird. An dem freien Ende des Befestigungselementes 63, 44 sind beispielsweise Verriegelungselemente 68, 69 in Gestalt abnehmbarer Klettelemente befestigt, die auf der Außenseite des Verriegelungselementes 63, 64 bzw. auf Flauschbereichen der Fixiereinrichtungen 66, 67 außen auf den medialen Taschen 62 festgelegt werden können. Die Festlegung an den Befestigungselementen 63, 64 und auf den Fixiereinrichtungen 66, 67 erfolgt reversibel. Die Befestigungselemente 63, 64 können flexibel und unelastisch, flexibel und elastisch sowie nur bereichsweise elastisch sein, ebenfalls ist es möglich, dass die das Verbindungselement 63, 64 reversibel an dem Befestigungsmittel 60 festgelegt ist, beispielsweise durch Klettverschlüsse.

Im angelegten Zustand, wie in der Figur 2 dargestellt, überbrückt das Verbindungselement 63, 64 den Freiraum innerhalb des schalenartigen Schienbeinteils20 von lateral nach medial. Das Schienbeinteil 20 kann eine Verformung der Vorsprünge 21 in Richtung aufeinander zu zulassen, so dass eine Anpassung an die Abmessungen der Gliedmaße, an die die Orthese anzulegen ist, erfolgen kann. Die Einstellung kann dabei über die über die Befestigungselemente 63, 64 aufgebrachte Umfangskraft erfolgen.

Die dargestellte Ausführungsform ist besonders vorteilhaft für Patienten, die halbseitig gelähmt sind oder eine halbseitige Schwächung aufweisen. Wird das Verbindungselement 63 von medial nach lateral geführt, gedehnt und unter Spannung auf einer lateralen Tasche 61 festgelegt, kann über die nicht von der Schwächung betroffene Hand eine sichere Festlegung der Orthese dadurch erfolgen, dass das Verbindungselement 63, 64 anschließend hinter der Gliedmaße hindurchgeführt und eine Verriegelung auf der Medialseite erfolgt.

Das Befestigungsmittel 60 kann auch als einteiliges Element oder als ein mehrteiliges, dauerhaft miteinander verbundenes Element ausgebildet sein, das eine Polsterfunktion aufweist. Die medialen und lateralen Vorsprünge 21 bilden autoadaptive Seitenflügel, die sich durch die Umfangskraft, die durch das Verbindungselement 63, 64 aufgebracht wird, selbstständig an die Form der Gliedmaße anpasst.

Durch Y-Klettbänder 68, 69 als Hakenbereiche ist es möglich, die als Gurte ausgeführten Befestigungselemente 63, 64 einfach zu kürzen, indem die Y-Klettbänder von den mit Flausch versehenden Befestigungselementen 63, 64 entfernt, die Länge auf das gewünschte Maß gekürzt und anschließend wieder aufgebracht werden.

Insbesondere bei Schlaganfallpatienten ist zu beachten, dass diese nur die nicht betroffene Körperseite zum Anziehen bzw. Anlegen der Orthese einsetzen können. Durch die laterale Anordnung der Befestigungselemente 63, 64 und die Führung von lateral nach medial zum Befestigen kann der Orthesennutzer im Sitzen mit der nicht betroffenen Seite das Verbindungselement 63, 64 von lateral greifen und einfach nach medial gezogen und dort an den dort versehenen Haken- und Flauschbereichen 66, 67 festgelegt werden.

## Patentansprüche

1. Knöchel-Fuß-Orthese mit einem Fußteil (10), das eine Sohle (11) zur Aufnahme eines Fußes aufweist, und einem Schienbeinteil (20), das sich im angelegten Zustand frontal an ein Schienbein anlegt und mit dem Fußteil (10) über eine Feder (30) verbunden ist, wobei die Feder (30) medial verläuft, **dadurch gekennzeichnet dass** die Feder (30) posterior zu einem Knöchelbereich (40) einen Gelenkbereich (35) ausbildet, der eine Verlagerung des Schienbeinteils (20) relativ zu dem Fußteil (10) in anterior-posterior-Richtung zulässt.

2. Knöchel-Fuß-Orthese nach Anspruch 1, **dadurch gekennzeichnet, dass** der Gelenkbereich (35) um eine proximal-distal-Achse torsionstabil ausgebildet ist.

3. Knöchel-Fuß-Orthese nach Anspruch 1 oder 2, **dadurch gekennzeichnet, dass** der Gelenkbereich (35) in medial-lateral-Richtung biegesteif ausgebildet ist,

4. Knöchel-Fuß-Orthese nach einem der voranstehenden Ansprüche, **dadurch gekennzeichnet, dass** die Feder (30) vom Fußteil (10) schräg nach dorsal oben verläuft und hinter dem Knöchelbereich (40) schräg nach ventral oben verläuft.

5. Knöchel-Fuß-Orthese nach einem der voranstehenden Ansprüche, **dadurch gekennzeichnet, dass** die Feder (30) eine Verjüngung (36) in anterior-posterior-Richtung aufweist.

6. Knöchel-Fuß-Orthese nach einem der voranstehenden Ansprüche, **dadurch gekennzeichnet, dass** die Feder (30) in anterior-posterior-Richtung ein geringeres Widerstandsmoment als in medial-lateral-Richtung aufweist.

7. Knöchel-Fuß-Orthese nach einem der voranstehenden Ansprüche, **dadurch gekennzeichnet, dass** die Feder (30) im Gelenkbereich (35) einen Materialaufbau aufweist, der eine in anterior-posterior-Richtung höhere Elastizität als in medial-lateral-Richtung hat.

8. Knöchel-Fuß-Orthese nach einem der voranstehenden Ansprüche, **dadurch gekennzeichnet, dass** das Schienbeinteil (20) rinnenförmig oder schalenförmig ausgebildet und in der Medialebene biegesteif ausgeführt ist.

9. Knöchel-Fuß-Orthese nach einem der voranstehenden Ansprüche, **dadurch gekennzeichnet, dass** das Schienbeinteil (20) in medial-Richtung und/oder lateral-Richtung elastisch ausgebildet ist.

10. Knöchel-Fuß-Orthese nach einem der voranstehenden Ansprüche, **dadurch gekennzeichnet, dass** die Feder (30) bezüglich einer Sagittalebene in Medialrichtung gewölbt ausgebildet ist.

11. Knöchel-Fuß-Orthese nach einem der voranstehenden Ansprüche, **dadurch gekennzeichnet, dass** das Schienbeinteil (20) eine Polsterung (65) aufweist.

12. Knöchel-Fuß-Orthese nach Anspruch 11, **dadurch gekennzeichnet, dass** die Polsterung (65) über Taschen (61, 62) an Vorsprüngen (21) oder Aufnahmebereichen (21, 22) festgelegt ist.

13. Knöchel-Fuß-Orthese nach Anspruch 11 oder 12, **dadurch gekennzeichnet, dass** die Polsterung (65) Befestigungselemente (63, 64) aufweist, die von lateral nach medial geführt sind.

14. Knöchel-Fuß-Orthese nach einem der voranstehenden Ansprüche, **dadurch gekennzeichnet, dass** die Feder (30) als Bogen ausgeführt ist.

15. Knöchel-Fuß-Orthese nach einem der voranstehenden Ansprüche, **dadurch gekennzeichnet, dass** die Feder (30) im Knöchelbereich (40) die geringste Steifigkeit gegen eine Biegung um eine Knöchelgelenksachse (50) aufweist.

## Claims

1. An ankle-foot orthosis with a foot part (10), which has a sole (11) for receiving a foot, and with a shin part (20) which, when fitted in place, bears on the frontal aspect of a shin and is connected to the foot part (10) via a spring (30), wherein the spring (30) extends medially, **characterized in that** the spring (30), posterior to an ankle area (40), forms a hinge area (35) that allows a movement of the shin part (20) relative to the foot part (10) in the anterior-posterior direction.

2. The ankle-foot orthosis as claimed in claim 1, **characterized in that** the hinge area (35) is designed to be torsionally stable about a proximal-distal axis.

3. The ankle-foot orthosis as claimed in claim 1 or 2, **characterized in that** the hinge area (35) is designed to be flexurally stiff in the medial-lateral direction.

4. The ankle-foot orthosis as claimed in one of the preceding claims, **characterized in that** the spring (30) extends obliquely upward in the dorsal direction from the foot part (10) and extends obliquely upward in the ventral direction behind the ankle area (40).

5. The ankle-foot orthosis as claimed in one of the preceding claims, **characterized in that** the spring (30) has a tapering (36) in the anterior-posterior direction.

6. The ankle-foot orthosis as claimed in one of the preceding claims, **characterized in that** the spring (30) has a lower section modulus in the anterior-posterior direction than in the medial-lateral direction.

7. The ankle-foot orthosis as claimed in one of the preceding claims, **characterized in that** the spring (30) has, in the hinge area (35), a material structure that has a greater elasticity in the anterior-posterior direction than in the medial-lat-eral direction.

8. The ankle-foot orthosis as claimed in one of the preceding claims, **characterized in that** the shin part (20) has a gutter shape or cup shape and is flexurally stiff in the medial plane.

9. The ankle-foot orthosis as claimed in one of the preceding claims, **characterized in that** the shin part (20) is elastic in the medial direction and/or lateral direction.

10. The ankle-foot orthosis as claimed in one of the preceding claims, **characterized in that** the spring (30) is curved in the medial direction with respect to a sagittal plane.

11. The ankle-foot orthosis as claimed in one of the preceding claims, **characterized in that** the shin part (20) has a padding (65).

12. The ankle-foot orthosis as claimed in claim 11, **characterized in that** the padding (65) is secured via pockets (61, 62) on projections (21) or receiving regions (21, 22).

13. The ankle-foot orthosis as claimed in claim 11 or 12, **characterized in that** the padding (65) has fastening elements (63, 64), which are guided from the lateral side to the medial side.

14. The ankle-foot orthosis as claimed in one of the preceding claims, **characterized in that** the spring (30) is designed as an arc.

15. The ankle-foot orthosis as claimed in one of the preceding claims, **characterized in that** the spring (30) in the ankle area (40) has the least stiffness against bending about an ankle-joint axis (50).

## Revendications

1. Orthèse de cheville-pied comportant une partie de pied (10) qui présente une semelle (11) pour recevoir un pied, et une partie de tibia (20) qui, à l'état appliqué, se place de face sur un tibia et est reliée avec la partie de pied (10) par l'intermédiaire d'un ressort (30), dans laquelle le ressort (30) s'étend en direction médiale, **caractérisée en ce que** le ressort (30), postérieurement à la zone de cheville (40), forme une zone d'articulation (35) qui permet un déplacement de la partie de tibia (20) par rapport à la partie de pied (10) en direction antérieure-postérieure.

2. Orthèse de cheville-pied selon la revendication 1, **caractérisée en ce que** la zone d'articulation (35) est réalisée stable à la torsion autour d'un axe proximal-distal.

3. Orthèse de cheville-pied selon la revendication 1 ou 2, **caractérisée en ce que** la zone d'articulation (35) est réalisée rigide à la flexion en direction médiale-latérale.

4. Orthèse de cheville-pied selon l'une des revendications précédentes, **caractérisée en ce que** le ressort (30) s'étend depuis la partie de pied (10) en oblique vers le haut du côté dorsal et, derrière la zone de cheville (40) en oblique vers le haut côté ventral.

5. Orthèse de cheville-pied selon l'une des revendications précédentes, **caractérisée en ce que** le ressort (30) présente un rétrécissement (36) en direction antérieure-postérieure.

6. Orthèse de cheville-pied selon l'une des revendications précédentes, **caractérisée en ce que** le ressort (30) présente en direction antérieure-postérieure un couple de résistance plus faible qu'en direction médiale-latérale.

7. Orthèse de cheville-pied selon l'une des revendications précédentes, **caractérisée en ce que** le ressort (30) présente dans la zone d'articulation (35) une structure de matériau qui a une plus grande élasticité en direction antérieure-postérieure qu'en direction médiale-latérale.

8. Orthèse de cheville-pied selon l'une des revendications précédentes, **caractérisée en ce que** la partie de tibia (20) est exécutée en forme de goulette ou de coque et réalisée rigide à la torsion dans le plan médial.

9. Orthèse de cheville-pied selon l'une des revendications précédentes, **caractérisée en ce que** la partie de tibia (20) est réalisée élastique en direction médiale et/ou latérale.

10. Orthèse de cheville-pied selon l'une des revendications précédentes, **caractérisée en ce que** le ressort (30) est réalisé bombé par rapport à un plan sagittal en direction médiale.

11. Orthèse de cheville-pied selon l'une des revendications précédentes, **caractérisée en ce que** la partie de tibia (20) présente un rembourrage (65).

12. Orthèse de cheville-pied selon la revendication 11, **caractérisée en ce que** le rembourrage (65) est fixé par l'intermédiaire de poches (61, 62) sur des saillies (21) ou des zones de réception (21, 22).

13. de cheville-pied selon la revendication 11 ou 12, **caractérisée en ce que** le rembourrage (65) présente des éléments de fixation (63, 64) qui sont guidés depuis la zone latérale vers la zone médiale.

14. Orthèse de cheville-pied selon l'une des revendications précédentes, **caractérisée en ce que** le ressort (30) est réalisé sous forme d'arc.

15. Orthèse de cheville-pied selon l'une des revendications précédentes, **caractérisée en ce que** le ressort (30) présente dans la zone de cheville (40) la plus faible rigidité à l'encontre d'une flexion autour d'un axe d'articulation de cheville (50).
